# EUROPEAN PATENT APPLICATION

(11) **EP 2 255 831 A1**
(43) Date of publication of application: **01.12.2010**
(21) Application number: 09290379.8
(22) Date of filing: 25.05.2009
(51) Int. Cl.: A61K 47/48, A61K 9/127, A61K 39/02

(54) **Liposome based diepitope constructs**

(71) Applicant: Institut Pasteur, 75015 Paris (FR)
(72) Inventor: Mulard, Laurence, 94270 Le Kremlin Bicetre (FR); Phalipon, Armelle, 75015 Paris (FR); Schuber, Francis, 67000 Strasbourg (FR); Said Hassane, Fatouma, 34090 Montpellier (FR); Frisch, Benoit, 67100 Strasbourg (FR)
(74) Representative: Thomas, Dean

(57) **Abstract**

The present invention relates to diepitope constructs which comprise a liposome, which in turn comprises a plurality of functionalized lipid anchors disposed upon a surface of the liposome. The construct also comprises a saccharide epitope, which comprises a first chemical attachment moiety and a peptide epitope, which comprises a second chemical attachment moiety. The saccharide epitope and said peptide epitope are not covalently attached to each other, but instead are covalently attached to the plurality of functionalized lipid anchors.

SEQUENCE LISTING

<110>  Institut Pasteur

<120>  Liposome based diepitope constructs

<130>  F226 -170 EP

<160>  10

<170>  PatentIn version 3.3

<210>  1
<211>  14
<212>  PRT
<213>  Artificial

<220>
<223>  HA 307-319 plus C terminus Cysteine residue

<400>  1

Pro Lys Tyr Val Lys Gln Asn Thr Leu Lys Leu Ala Thr Cys
1               5                   10


<210>  2
<211>  15
<212>  PRT
<213>  Artificial

<220>
<223>  PADRE Th epitope


<220>
<221>  cyclohexyalanine
<222>  (3)..(3)

<220>
<221>  amino caproic acid
<222>  (14)..(14)

<400>  2

Ala Lys Xaa Val Ala Ala Trp Thr Leu Lys Ala Ala Ala Xaa Cys
1               5                   10                  15


<210>  3
<211>  15
<212>  PRT
<213>  Artificial

<220>
<223>  TT830-843 Th epitope

<400>  3

Gln Tyr Ile Lys Ala Asn Ser Lys Phe Ile Gly Ile Thr Glu Leu
1               5                   10                  15


<210>  4
<211>  15
<212>  PRT
<213>  Artificial

<220>
<223>  TT1084-1099 Th epitope

<400>  4

Val Ser Ile Asp Lys Phe Arg Ile Phe Cys Lys Ala Asn Pro Lys
1               5                   10                  15


<210>  5
<211>  16
<212>  PRT
<213>  Artificial

<220>
<223>  TTl 174-1189 Th epitope

<400>  5

Leu Lys Phe Ile Ile Lys Arg Tyr Thr Pro Asn Asn Glu Ile Asp Ser
1               5                   10                  15


<210>  6
<211>  16
<212>  PRT
<213>  Artificial

<220>
<223>  TT 1064-1079

<400>  6

Ile Arg Glu Asp Asn Asn Ile Thr Leu Lys Leu Asp Arg Cys Asn Asn
1               5                   10                  15


<210>  7
<211>  21
<212>  PRT
<213>  Artificial

<220>
<223>  TT947-967 Th epitope

<400>  7

Phe Asn Asn Phe Thr Val Ser Phe Trp Leu Arg Val Pro Lys Val Ser
1               5                   10                  15


Ala Ser His Leu Glu
            20


<210>  8
<211>  20
<212>  PRT
<213>  Artificial

<220>
<223>  Neisseria meningtidis Th epitope

<400>  8

Tyr Ala Phe Lys Tyr Ala Arg His Ala Asn Val Gly Arg Asn Ala Phe
1               5                   10                  15

Glu Leu Phe Leu
            20

<210>  9
<211>  18
<212>  PRT
<213>  Artificial

<220>
<223>  P. falsiparum Th epitope

<400>  9

Glu Lys Lys Ile Ala Lys Met Glu Lys Ala Ser Ser Val Phe Asn Val
1               5                   10                  15

Asn Asn

<210>  10
<211>  13
<212>  PRT
<213>  Artificial

<220>
<223>  Polio virus Th epitope

<400>  10

Lys Leu Phe Ala Val Trp Lys Ile Thr Tyr Lys Asp Thr
1               5                   10

## Description

The present invention relates to liposome based diepitope constructs which comprise at least one saccharide B cell epitope and at least one Th cell epitope, wherein these epitopes are not covalently linked. The present invention also relates to medicaments and vaccines comprising diepitope liposome based constructs as well as to methods of synthesizing such diepitope liposome constructs.

Polysaccharide conjugation to carrier proteins, allows the efficient conversion of bacterial polysaccharide antigens into T cell-dependent antigens triggering long-term immunity and immune memory and constitutes a great achievement of the last two decades in the field of vaccine research. Nevertheless, in spite of successes in developing vaccines against bacterial infections for use with infants and young children [1, 2], this approach has some limitations. Despite numerous improvements in polysaccharide production and polysaccharide/protein conjugation, matching the quality control requirements of a composition which must be mass produced and administered to amongst others children and other vulnerable patient groups such as the elderly, continues to present significant problems. Partial alteration of the polysaccharide which may occur during the required chemical transformation steps and this can lead to the partial loss or divergence in immunogenicity of the final construct. Such effects may be even more pronounced when dealing with lipopolysaccharide (LPS) antigens than with capsular polysaccharide antigens.

Recent progress in glycochemistry and improved knowledge about the basis of the protective immune response has encouraged the investigation of alternative solutions to these problems. Thus, synthetic oligosaccharide-protein conjugates, involving functional mimics of the natural polysaccharide antigens, have emerged as an attractive alternative option [3]. This strategy was recently validated in humans resulting in the licensing of Quimi Hib^{®} against *Haemophilus influenzae* type b, the first vaccine in this category [4]. However, in addition to T-helper (Th) epitopes which contribute to the required T-independent to T-dependent conversion of the immune response, the carrier proteins also provide their own B-cell epitopes. As a consequence, an important part of the immune response is often directed towards the carrier, possibly resulting in carrier-induced epitope suppression [5].

To avoid such epitope competition, the use of chemically defined synthetic vaccines represents an attractive alternative vaccination strategy. In this approach, Th peptides/antigens of natural or artificial origin are used alone or in combination. For example, such peptides are currently under investigation as suitable sources of the T-cell help required for efficient vaccination with carbohydrate antigens [6]. Additionally, in the search for alternative strategies, nanoparticulate systems are also of interest and could offer some advantages over proteins as vaccine carriers [7].

In this respect, liposomes are regarded as attractive nanocarriers for the multivalent presentation of selected synthetic carbohydrate haptens, Th peptide epitopes and potent adjuvants. These versatile biocompatible (phospho)lipid vesicles are characterized by a low intrinsic immunogenicity and toxicity. They can carry multiple antigens either encapsulated, surface-bound or associated with the bilayers [8, 9], in addition to combinations with various adjuvants such as ligands of Toll-like receptors (TLR), these adjuvants can be either amphiphilic such as lipopeptides (e.g. Pam₃C and MALP-2 derivatives) and monophosphoryl lipid A [10-12] or hydrosoluble (e.g. CpG derivatives) [67, 68].

In addition to the above properties, liposomal constructs can be efficiently captured and endocytosed by antigen presenting cells (APCs) [13-15], this process being followed by antigen processing and presentation of the Th and/or CTL epitopes via MHC class II/I-dependent pathways. Importantly, clusters of B-cell epitopes exposed on the surface of the liposomes efficiently target antigen specific B lymphocytes by multivalent interactions with the B-cell antigen receptors (BCR), increasing the potency of the humoral response [16].

The inventors have previously designed liposomal dipeptide epitope vaccination constructs, in which two peptide epitopes (Th, B or CTL) are conjugated to the surface of preformed small unilamellar vesicles containing synthetic amphiphilic adjuvants [12]. These earlier liposome diepitope vaccination constructs triggered potent humoral [17] and anti-tumoral [18] immune responses. The conjugation steps involved high-yielding reactions between the peptides, modified with a cysteine residue either at their N- or C-terminus and thiol-reactive anchors incorporated into the bilayers of the vesicles.

Other workers in this field [41, 42, 62] have generated liposome constructs comprising a carbohydrate (B epitope) and peptide (Th epitope) by generating a consolidated glycolipopeptide molecule which comprises a carbohydrate B epitope and a peptide T epitope covalently linked to each other and which is also lipidated so that the entire molecule can self assemble into a liposome or vesicle.

The workers who developed these consolidated glycolipopeptide B/Th epitope constructs believed it was necessary in order to elicit the required humoral response and cellular response to covalently link the B and Th epitopes in the form of a glycopepetide, one common form in which saccharide epitopes are presented *in vivo.* The workers drew this conclusion as following vaccination with a first set of constructs, wherein a tumor saccharide B epitope (Le^{Y}) and a universal Th epitope (QYI) were not covalently linked, no anti-Le^{Y} antibodies were detected.

Significant problems exist with these types of consolidated B/Th molecules, in particular when working at a scale large enough to supply sufficient quantities of the consolidated B/Th molecule for use in the production of a vaccine, the final glycolipopeptide can be very difficult to generate due to low yields of the lipidation reaction, as the final glycolipopeptide is hydrophobic and/or amphiphilic. These high levels of hydrophobicity also cause difficulties in handling and storing the glycolipopeptides and finally the assembly of these into suitable liposome vesicles leads to a further loss in production. It is also impossible in the case where separate lipidated saccharide and lipidated peptide molecules are made, to determine what the final composition of the liposomes/vesicles will be. Therefore the production and handling of these glycolipopeptides on a large enough scale to allow the mass production of a vaccine is not presently possible.

Seeing these problems with the generation of anti-carbohydrate/peptide antibodies the inventors chose to investigate the feasibility and potency of a new strategy they have developed for the creation of innovative constructs comprising a peptide and a saccharide epitope presented upon the surface of a liposome.

According to a first aspect of the present invention there is provided a diepitope construct comprising:
a liposome, comprising a plurality of functionalized lipid anchors disposed upon a surface thereof;
a saccharide epitope, comprising a first chemical attachment moiety;
a peptide epitope, comprising a second chemical attachment moiety and wherein said saccharide epitope and said peptide epitope are not covalently attached to each other;
wherein said saccharide epitope and said peptide epitope are covalently attached to said plurality of functionalized lipid anchors.

The inventors have developed a new type of diepitope construct which comprises at least one saccharide and one peptide epitope linked to anchors present in a liposome, the entire liposome construct therefore acts as a vehicle for the induction of an immune response against both epitopes.

In the present Patent Application a liposome is a spherical structure which in particular comprises a phospholipid bilayer. Various other types of liposomes are known in the art and are encompassed by the present Patent Application.

In the present Patent Application a functionalized lipid anchor is a molecule which is stable in the phospholipid bilayer of a liposome and which comprises a specific groups of atoms (functional group) that are responsible for the characteristic chemical reactions of the lipid anchor with the first and second chemical attachment moiety. The same functional group will undergo the same or similar chemical reaction(s) regardless of the size of the molecule it is a part of. The functionalized lipid anchors according to the present invention can comprise more than one functional group with the same or a different group of atoms and/or chemical properties.

Diepitope constructs according to the present invention can comprise more than two epitopes and in particular can comprise a plurality of different saccharide and/or peptide epitopes allowing multi-epitope constructs to be generated. Each of these additional epitopes being characterised in that they comprise at least one chemical attachment moiety via which they can be covalently bonded to the plurality of lipid anchors.

The inventors have shown that constructs according to the present invention are efficient systems for the presentation of complex synthetic saccharides resulting in the induction of a strong and long-lasting protective humoral immune response against these saccharide epitopes and the pathogens/cell types from which they are derived.

The covalent association of epitopes upon the outer surface of preformed liposomes was an option proposed but untested in the art for saccharide epitopes. The ability to generate such saccharide/liposome constructs was desired, as previous experiments with liposome/peptide epitope constructs, had shown that irrespective of the administration mode, superior humoral immune responses are observed when peptide antigens are surface-linked rather than encapsulated [47, 48].

By 'preformed' the inventors mean that the liposome component of diepitope constructs according to the present invention were existing molecular entities prior to the bonding of these liposomes to the other specified components e.g. the saccharide and peptide epitopes.

The inventors, using the important pathogenic organism *Shigella flexneri* serotype 2a (SF2a) as a model, have shown that synthetic versions of saccharide epitopes from this pathogen, linked covalently to suitable liposomes via a plurality of lipid anchors, together with a peptide epitope can induce an initial immune response and long term immunity against this saccharide epitope and the *Shigella* pathogen. By generating these saccharide epitopes synthetically rather than using isolated microbial materials, this allows an even higher level of control over the final diepitope construct.

*Shigella* lipopolysaccharide (LPS) is a major surface antigen of the bacterium. The corresponding O-SP domain (O-SP), a polysaccharide defined by its repeating unit (RU), is both an essential virulence factor and the target of the infected host's protective immune response [56, 57].

In particular the inventors have used a saccharide derived from the O-SP domain of the formula AB(E)CD, the RU, wherein A is an α-L-rhamnopyranosyl-(1,2) residue, B is an α-L-rhamnopyranosyl-(1,3) residue, C is an α-L-rhamnopyranosyl-(1,3) residue, E is an α-D-glucopyranosyl-(1,4) residue, D is a 2-acetamido-2-deoxy-β-D-glucopyranosyl-(1,2) residue. The inventors have used this saccharide either as a monomer or a trimer and have seen significantly better results using the trimeric form as a saccharide hapten.

In contrast to other findings [42, 62] in the art, the data presented herein show also that covalent linking of B-cell epitopes with Th epitopes is not an absolute requirement for inducing a strong IgG-mediated immune response against SF2a LPS as it was previously thought to be.

Indeed, the immune response following administration of the diepitope constructs according to the present invention and in particular the response observed with the pentadecasaccharide construct is characterized by a very low IgM/IgG ratio and a prevalence of IgG1 and IgG3 subtypes in boosted mice. The Th2 type- nature of the response, attested by the presence of IgG1 subclass, agrees with earlier studies on humans either naturally exposed to or vaccinated against SF2a, showing a predominant IgG2-mediated Th2 antibody response.

A likely interpretation of these results is that the oligosaccharides displayed at the surface of one of the constructs developed by the inventors favor a strong interaction with the BCR, triggering the uptake of the glycoliposomes by the B-cell, and subsequent processing towards B and T cells cooperation. In contrast to previous studies, it is noteworthy that a concomitant T cell independent (TI-2 antigen) response was induced as attested by the production of IgG3. This observation is best explained by the efficient liposome-mediated multivalent presentation of the oligosaccharides to BCRs resulting in their clustering and B cell activation. This process is most probably favored by a correct orientation of the exposed oligosaccharides within a fluid liposomal membrane, initiating a T cell-independent immune response before liposome uptake by B-cells and inception of the T cell help.

As the inventors have now shown that it is not necessary to covalently link a saccharide to a peptide epitope as previously thought this presents the opportunity to easily generate entirely new classes of immunogens which for instance comprise different relative quantities of one epitope in comparison to another for instance the ratio of saccharide epitope to peptide epitope. This can easily be achieved as the different epitope components of the constructs are not linked and hence can be coupled with the liposome at independently determined concentrations. Likewise constructs comprising liposomes which comprise a mixture of different epitopes at different concentrations or densities per liposome can be easily made as each of these epitopes can be independently attached to the liposome.

In particular the saccharide epitope is microbial.

In the present invention a microbial saccharide epitope is intended to mean any natural or synthetic version of an epitope from any microorganism, including parts thereof such as coat molecules as well as saccharides from specific life stages of the microorganism such as spores. In particular the microorganism may be prokaryotic, eukaryotic or a virus.

Various important saccharide epitopes are known in the art for instance from prokaryotes such as *Salmonella enteritidis, Streptococcus pneumoniae, Haemophilus influenzae, Neisseria meningitidis*; other micro organisms such as viruses including HIV and hepatitis C, fungi [58] and eukaryotic parasites [59].

In addition to microbial saccharide epitopes, altered saccharide antigens are associated with some cancer/proliferative disease cells [60] and the present invention therefore also comprises constructs with 'self' carbohydrate epitopes.

In particular the saccharide epitope is a B cell epitope and the peptide epitope is a Th cell epitope.

A B cell epitope is an antigen or a fragment thereof which is recognized and bound by a B-cell receptor.

A Th cell epitope is an antigen or a fragment thereof which is recognized and bound by a T-cell receptor.

The processing and presentation of different epitopes by the various receptors of the immune system, allows the body to recognise, modulate and memorise the various antigens to which the immune system is exposed.

As explained above in order to elicit a good and prolonged response to a saccharide epitope it is necessary to stimulate both a B cell response to the saccharide epitope as well as to stimulate the conversion of this response from a T cell independent to T cell dependent response by stimulating a T helper cell response. In the present work the inventors have validated the new set of diepitope constructs using the universal Th epitope HA 307-319 (PKY), a "universal" Th epitope derived from influenza virus hemagglutinin (HA) [69].

Many other Th epitopes are known in the art and these are encompassed by the present invention. Other examples of suitable Th epitopes include the PADRE peptide, AKXVAAWTLKAAAZC (X = L-cyclohexylalanine, Z = aminocaproic acid) (SEQ ID NO: 2), including all the analogues described in [66]; as well as peptides derived from tetanus toxin, e.g., (TT830-843); QYIKANSKFIGITEL (SEQ ID NO: 3), (TT1084-1099) VSIDKFRIFCKANPK (SEQ ID NO: 4), (TT 1174-1189) LKFIIKRYTPNNEIDS (SEQ ID NO: 5), (TT 1064-1079) IREDNNITLKLDRCNN (SEQ ID NO: 6), and (TT947-967) FNNFTVSFWLRVPKVSASHLE (SEQ ID NO: 7). Peptides derived from polio virus, e.g., KLFAVWKITYKDT (SEQ ID NO: 10); Peptides derived from *Neisseria meningitidis,* e.g., YAFKYARHANVGRNAFELFL (SEQ ID NO: 8); and Peptides derived from *P. falciparum* CSP, e.g., EKKIAKMEKASSVFNVNN (SEQ ID NO: 9).

In addition to B cell and Th cell epitopes, diepitope constructs according to the present invention may also comprise CTL (cytotoxic T cell) epitopes and/or epitopes which are recognised and presented as both B/Th/CTL epitopes.

In particular the saccharide epitope is synthetic.

By synthetic it is intended to mean a molecule which structurally and/or functionally mimics in whole or part a natural epitope/antigen but which was made either in part or completely from materials other than the natural epitope/antigen.

In addition to the saccharide epitope, the peptide and all other components of the diepitope construct according to the present invention may also be synthetic.

In particular the saccharide epitope comprises multiple saccharide units.

Saccharides *in vivo,* for example bacterial surface polysaccharides, may comprise multimerized versions of a single or more commonly several saccharide residues, termed repeating units. Therefore a saccharide epitope according to the present invention may comprise multimerized saccharide repeating units.

In the present Patent Application by saccharide units it is intended to mean any organic compound that is an aldehyde or a ketone which comprises many hydroxyl groups added, usually one on each carbon atom that is not part of the aldehyde or ketone functional group. The most basic saccharides are called monosaccharides, such as glucose, galactose, and fructose. Vastly more complex saccharides, possibly modified by a range of non carbohydrate substituants, are known in nature and have been generated in the laboratory; all such saccharide units are encompassed by the present Patent Application.

In particular the saccharide epitope is a polysaccharide.

The inventors when comparing the performance, that is the immune response elicited by the various constructs, of a *Shigella* derived saccharide epitope (single copy) versus a trimerized version of the same saccharide epitope (three copies) have shown that polysaccharide epitopes appear to elicit a stronger immune response. The inventors believe these differences may be due to the size of the saccharide epitope having an effect upon the immune response elicited.

In particular the first chemical attachment moiety on the saccharide epitope comprises a thiol precursor group.

In addition to the use of thiol precursor groups, the first chemical attachment moiety can be any high yielding conjugation reaction compatible with aqueous media. For example a Staudinger reaction [63], click-chemistry [64], activated acid [70]. In addition according to the present invention each epitope may comprise more than one chemical attachment moiety thereby increasing the yield of multi-epitope liposome based constructs.

In particular the saccharide or the polysaccharide epitope according to the present invention, may comprise a spacer arm between said polysaccharide epitope and said first chemical attachment moiety.

In order to elicit as specific an immune response as possible, the inventors have found that it can be necessary to separate the saccharide epitope from the first chemical attachment moiety via a spacer arm such as an ethyl spacer. This spacer can also be larger and may in particular comprise a linear or branched carbon chain of three or more carbon atoms.

The inventors have found that it is possible to attach a thiol precursor group to a saccharide epitope of interest. This thiol precursor group allows, following reduction, the chemical attachment of the saccharide via the thiol group to a thiol-reactive group such as a maleimide group present upon the liposome anchor.

Other examples of thiol reactive groups include bromoacetyl, iodoacetyl, allyl and any other chemical groups which can form disulfide bonds such as by native ligation [65].

In particular the functionalized liposome anchor may comprise more than one functional group.

The inventors have shown that in addition to the binding of the specified epitopes to separate lipid anchors both epitopes may also be bound by the same lipid anchor, wherein this anchor comprises more than one functional group.

In particular the plurality of functionalized liposome anchors comprise a thiol-reactive functional group.

In particular the thiol-reactive functional group is a maleimide group.

In particular therefore the plurality of lipid anchors are adjuvants.

Most particularly the plurality of lipid anchors consist of S-[2,3-bis(palmitoyloxy)-2(R)-propyl]-N-palmitoyl-(R)-cysteinyl-alanyl-glycine-maleimide (Pam₃CAG-Mal).

Many other suitable lipid anchors exist such as Pam₃-Cysteine-Serine-Serine-Mal (Pam₃CSS-Mal) [61] and also such suitable lipid anchors such as functionalized phospholipids, cholesterol or fatty acid anchors with no adjuvant properties which can be positioned in a liposome. These other lipid anchors must comprise a functionalized group to which the chemical attachment moiety of a saccharide epitope can be attached, in addition the liposome may also comprise non-functionalized adjuvants, that is molecules with adjuvant properties which are contained within the liposome but do not contain a functionalized group and hence do not bind to the saccharide epitope.

The choice of functionalized Pam₃CAG-Mal in the inventors constructs is of importance. Previously this lipid anchor, either co-administered or covalently linked to peptide antigens, has been shown to be a strong adjuvant and to trigger intense and specific immune responses.

The combination of this known adjuvant, Pam₃CAG-Mal, in the present invention with a saccharide B cell epitope is the first time it has been shown that this combination of immunogens is able to act in concert so as to elicit an immune response.

Alternatively the plurality of lipid anchors are not adjuvants.

By providing a first population of lipid anchors to perform the function of attaching the various epitopes to the liposome and a second population of lipid anchors which have adjuvant properties, this allows the constructs according the present invention to be even more varied in terms of the density of epitopes and adjuvant molecules per liposome.

In particular the second chemical attachment moiety of the peptide epitope comprises at least one cysteine residue. The cysteine residue can be positioned at either the C- or N- terminus of the peptide epitope or alternatively may be positioned within the peptide so long as the attachment of residue to an internal cysteine residue.

Alternatively the second attachment moiety may comprise a thiol-functionalized linker, positioned either at the C- or N-terminus.

In addition to coupling the saccharide epitope to the plurality of functionalized lipid anchors via a thiol-reactive group, it is also possible to attach the peptide epitope to another or the same plurality of functionalized lipid anchors present in the same liposome via a similar chemical mechanism.

Alternatively the saccharide and peptide epitopes may be attached to the plurality of functionalized lipid anchors via different chemical reactions.

According to a second aspect of the present invention there is provided a method to prepare a diepitope liposome construct according to the first aspect of the present invention, comprising the steps:
a. preparing at least one set of liposomes, wherein each liposome comprises at least two liposome anchors;
b. preparing at least one set of saccharide epitopes and at least one set of peptide epitopes;
c. chemically activating said liposome anchors and said epitopes from steps a.;
d. mixing said liposomes and said epitopes from step c;
e. chemically activating said epitopes from step b and mixing with said liposomes from step d.

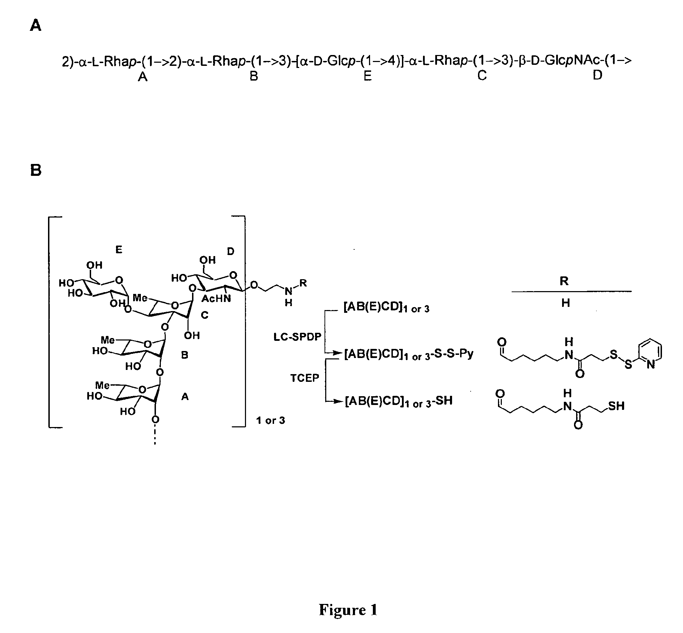

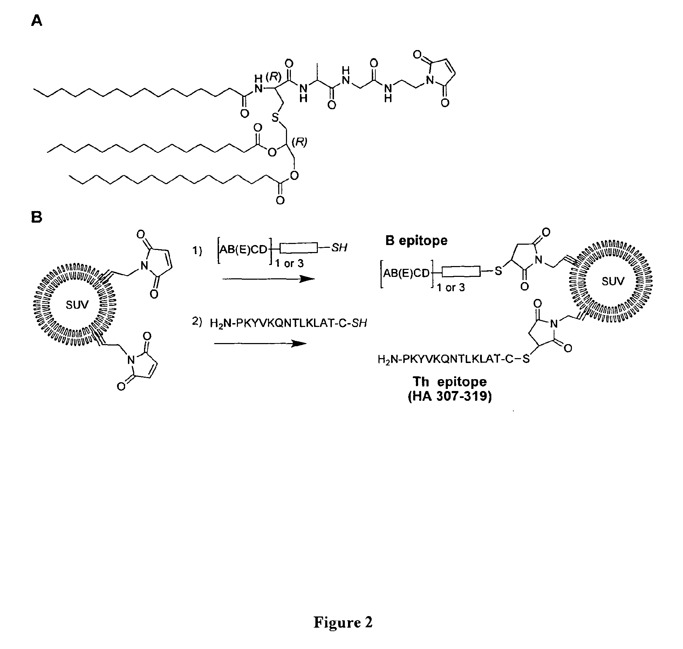

The inventors method of bioconjugating synthetic thiol-functionalized carbohydrate B-cell epitopes and Th peptides to lipopeptide adjuvants so as to generate novel diepitope constructs is based on an advantageous methodology they have developed, which has not been reported before. The coupling of synthetic thiol-functionalized carbohydrate B-cell epitopes and Th peptides to lipopeptide adjuvants were performed independently (Figs. 1 and 2), on the same preformed liposome, using a high-yielding chemoselective ligation procedure in aqueous media (Fig. 2B).

Thus, diepitope constructs according to the present invention have the following advantages: (i) the oligosaccharide B-cell epitopes, displayed on the liposome surface, are uniformly presented in an oriented fashion preserving their structural integrity; and (ii) the densities of epitopes exposed at the liposome surface are efficiently controlled.

Moreover, from a chemical standpoint, this simple, quite versatile and controllable conjugation protocol on preformed liposomes allows a worker to circumvent the rather cumbersome prior art need to synthesise, purify and handle lipidated oligosaccharides and peptides [49] and/or lipidated glycopeptides, and also avoids potential losses during the formulation step. Importantly, this convergent process, which relies on a common adjuvant anchor-containing liposome, offers an easy access to diversity in terms of B-cell and Th epitope composition.

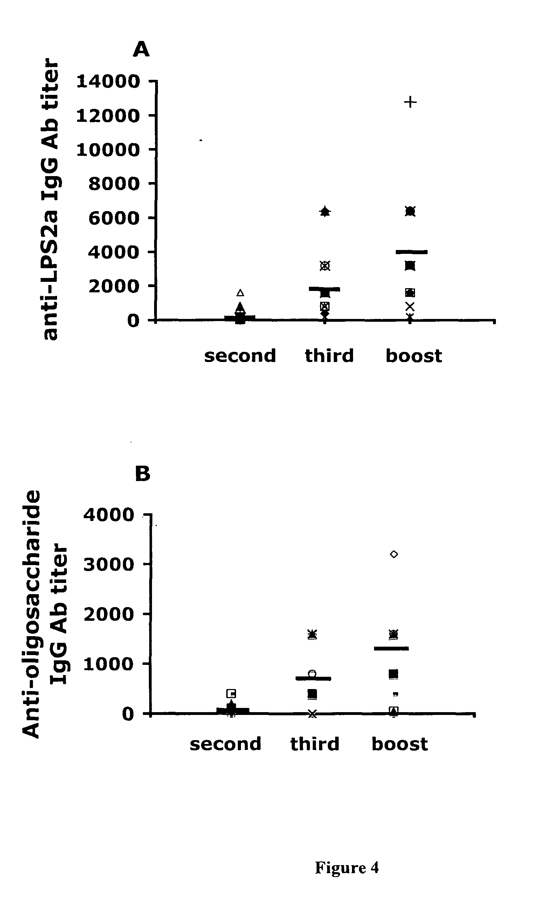

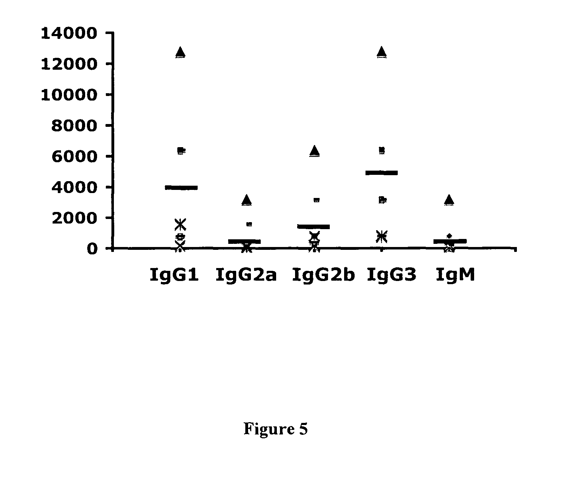

The immunogenicity of the diepitope glycoliposomal constructs developed and validated herein (Figs. 4 and 5) demonstrates the utility of this approach.

The inventors have found that the order of steps in the attachment of epitopes to the functionalized lipid anchors is important; in particular the inventors have found that the coupling of the peptide epitope under certain conditions can cause an immediate aggregation of the liposomes, this probably being due to the hydrophobic nature of the peptide epitope used. The inventors have determined that by first coupling the oligosaccharide to the lipid anchor, that this maintains the surface of the liposome in a polar state, even following addition of the peptide and so prevents aggregation.

According to a third aspect of the present invention there is provided a saccharide of the formula AB(E)CD-SH, wherein A is an α-L-rhamnopyranosyl-(1,2) residue, B is an α-L-rhamnopyranosyl-(1,3) residue, C is an α-L-rhamnopyranosyl-(1,3) residue, E is an α-D-glucopyranosyl-(1,4) residue, D is a 2-acetamido-2-deoxy-β-D-glucopyranosyl-(1,2) residue and -SH is a thiol functional group.

According to a further aspect of the present invention there is provided a polysaccharide [AB(E)CD]ₙ-SH, wherein said polysaccharide comprises at least three copies of the saccharide AB(E)CD wherein A, B, C, D, E and -SH are as defined previously and n is any integer equal to or greater than two and preferably greater than or equal to three.

The inventors identified the synthetic pentadecasaccharide [AB(E)CD]₃ representing three biological repeating units (Fig. 1) of SF2a O-SP as an efficient structural and functional SF2a O-SP mimic.

In particular the inventors have identified two SF2a synthetic oligosaccharides (B-cell epitopes), pentasaccharide AB(E)CD and pentadecasaccharide [AB(E)CD]₃, respectively. The inventors have shown that when these B-cell and Th epitopes (such as HA 307-319 (PKY)) were covalently conjugated to the surface of preformed liposomes via the maleimide functionalized lipopeptide anchor Pam₃CAG-Mal, these synthetic liposomal construct carrying the pentadecasaccharide [AB(E)CD]₃ induced a specific, long-lasting and protective immune response against SF2a, when administered intra muscularly to BALB/c mice.

In particular the saccharide or the polysaccharide according to the present invention, comprise an aminoethyl spacer arm between said polysaccharide epitope and said thiol functional group.

In their work with the Shigella antigen the Inventors have found that it is important as far as possible to not affect the properties of the 'D' saccharide to which the thiol group is attached and therefore to reduce the effects if this group both before and more importantly after its reaction with the lipid anchor they have used a spacer and in particular a aminoethyl spacer. This spacer can also be considerably larger and may in particular comprise a propyl, butyl or longer linear or branched carbon chain.

According to a fourth aspect of the present invention there is provided the use of a diepitope construct according to a first aspect of the present invention, or made using a method according to a second aspect of the present invention or a saccharide according to a third aspect of the present invention, for the preparation of a medicament.

Diepitope constructs according to the present invention represent a new class of fully synthetic multi-component vaccine, wherein epitopes and adjuvants are co-delivered to B lymphocytes within a single multivalent delivery system, a strategy which is known to greatly improve the induction of an efficient immune response

This class of new multivalent constructs and substances derived there from are therefore ideal for formulation as medicaments or therapeutic agents for the prevention and/or treatment of a disease caused by a pathogen or a condition in which the agent of pathology expresses a specific saccharide antigen, a component of which is comprised within the diepitope construct.

According to a fifth aspect of the present invention there is provided a vaccine comprising a liposome according to the first aspect of the present invention, or made using a method according to the second aspect of the present invention or a saccharide consisting of the formula AB(E)CD-SH, wherein A is an α-L-rhamnopyranosyl-(1,2) residue, B is an α-L-rhamnopyranosyl-(1,3) residue, C is an α-L-rhamnopyranosyl-(1,3) residue, E is an α-D-glucopyranosyl-(1,4) residue, D is a 2-acetamido-2-deoxy-β-D-glucopyranosyl-(1,2) residue and -SH is a thiol functional group.

The medicament or vaccine according to the present invention may be administered by any suitable means such as by topical, enteral or parenteral means.

In particular the medicament or vaccine may be administered via parental injection or infusion and most particularly by intramuscular injection.

For a better understanding of the invention and to show how the same may be carried into effect, there will now be shown by way of example only, specific embodiments, methods and processes according to the present invention with reference to the accompanying drawings in which:
**Fig. 1****. A -** Basic structure of the repeating unit of the O-specific polysaccharide moiety of *Shigella flexneri* 2a LPS. **B -** Chemical structure of the synthetic oligosaccharides [AB(E)CD]₁ or ₃ used as B-cell epitopes. The aminoethyl spacer arms were functionalized with Sulfo-LC-SPDP to introduce a thiol-precursor, which is easily reduced with TCEP into the required free thiol prior to conjugation to liposomes.
**Fig. 2****. A -** Structure of the functionalized lipopeptide anchor (*R,R*)-Pam₃CAG-Mal. **B** -Preparation of the oligosaccharide/peptide diepitope liposomal constructs. The coupling reaction of the thiol-bearing AB(E)CD, or [AB(E)CD]₃ haptens, respectively, and the cystein-modified Th peptide epitope with the maleimide function of Pam₃CAG-Mal incorporated in preformed liposomes was performed, in that order, at pH 6.5 in a 10 mM Hepes buffer containing 145 mM NaCl.

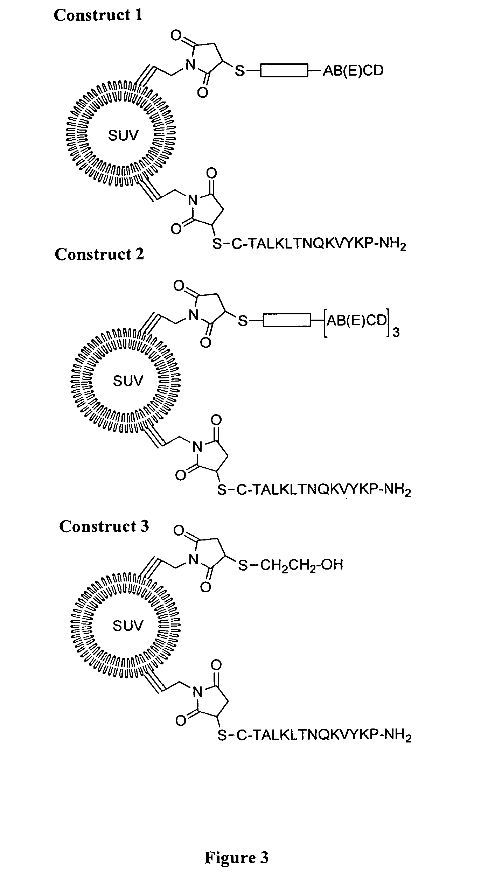

**Fig. 3****.** Glycoliposomal constructs tested for their immunogenicity in BALB/c mice. In Constructs **1** and **2,** the polysaccharide B-cell epitopes (respectively penta- and pentadecasaccharide) and HA 307-319 Th peptide epitope were conjugated to the outer surface of preformed liposomes by reaction with a functionalized lipopeptide adjuvant (*R*,*R*)-Pam₃CAG-Mal anchor. The control Construct **3** contains the Th epitope and a similar proportion of accessible anchor reacted with 2-mercaptoethanol. The respective proportions, in mol%, of the B-cell and Th epitopes relative to phospholipids are: Construct **1** (2.5:2.5), Construct **2** (0.83:2.5) and Construct **3** (2.5:2.5).
**Fig. 4****.** Immunogenicity of glycoliposomes carrying the pentadecasaccharide [AB(E)CD]₃. BALB/c mice were immunized i.m. with Construct **2** using 10 µg of pentadecasaccharide per dose and per animal. The IgG titers raised against SF2a LPS **(A)** and the [AB(E)CD]₃ oligosaccharide **(B)** were determined 7 days after the second and third immunization, and after the last boost. Individual IgG responses are shown and the mean Ab titers are represented by a horizontal bar. The results are representative of two independent experiments including 14 and 15 mice each.
**Fig. 5****.** Anti-LPS 2a IgM and IgG subclasses induced by Construct **2.** IgG1, IgG2a, IgG2b, IgG3 and IgM Ab titers against SF2a LPS were determined using nine sera giving the highest anti-LPS 2a IgG Ab titers after the boost. Individual responses are shown and the mean Ab titers are represented by a horizontal bar.

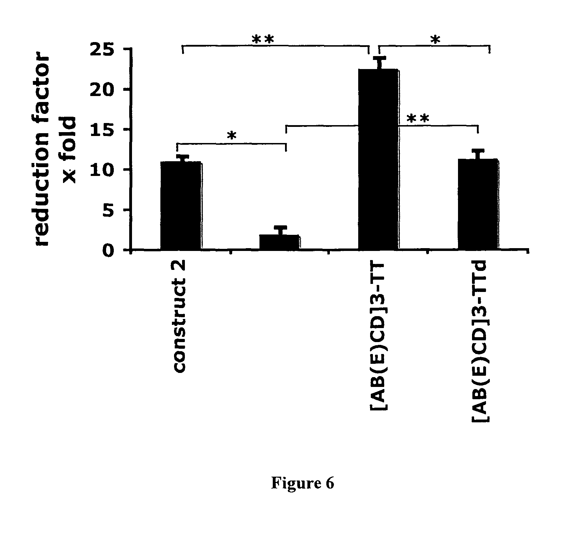

**Fig. 6**. Protection conferred by anti-LPS 2a IgG Abs induced with Construct 2. Naive mice were passively i.n. administered with immune or preimmune sera previously incubated with 10⁶ CFU of virulent SF2a bacteria. Mouse sera exhibiting a similar anti-LPS 2a Ab titer (1/6,400) were selected from each group of mice immunized with Construct **2** or [AB(E)CD]₃-TT. Measurement of CFU/lungs was performed 24 h post-infection. A reduction factor was determined which corresponds to the ratio between CFU/lungs in naive mice passively administered with pre-immune sera to that of naive mice passively administered with immune sera. « d » indicates a 10-fold dilution of serum before its incubation with SF2a bacteria. Data are representative of three independent experiments including seven mice per group for each serum tested. **p* value <0.05 (Student's *t* test) when comparing pure and diluted sera induced by Construct **2** or [AB(E)CD]₃-TT. ***p* value <0.05 (Student's *t* test) when comparing Construct **2** and [AB(E)CD]₃-TT.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example 1. Materials and methods

Egg yolk L-α phosphatidylcholine (PC), L-α phosphatidylglycerol (PG), transesterified from egg PC, and cholesterol (Chol) recrystallized in methanol were purchased from Sigma (St Quentin-Fallavier, France). The biologically active enantiomer *S*-[2,3-bis(palmitoyloxy)-2(*R*)-propyl]-*N*-palmitoyl-(*R*)-cysteinyl-alanyl-glycine (Pam₃CAG), prepared in the inventors laboratory using R-(+)-glycidol as chiral synthon [31], was converted into the maleimide derivative Pam₃CAG-Mal by reaction with 1-(2-aminoethyl)-pyrrole-2,5 dione as described previously [17].

### 1.1. Synthetic oligosaccharides functionalized for coupling

Sulfo-LC-SPDP (Pierce Chemicals, Interchim, Montluçon, France) (3.6 mg, 1.2 equiv) was added to a solution of synthetic AB(E)CD bearing an aminoethyl aglycon [22] (5 mg, 5.8 µmol) in 500 µL PBS, pH 7.3, and the mixture was stirred for 30 min at room temperature. RP-HPLC purification of the crude material (C₁₈ 5 µm 100 Å Kromasil AIT column, 10x250 mm, λ 230 nm) eluted with a 0-30% linear gradient over 20 min of CH₃CN in 0.01 M aq. TFA at 5.5 mL.min⁻¹, followed by freeze-drying, gave [AB(E)CD]-S-S-Py (6.1 mg, 89%) as a lyophilized powder. Rt (HPLC): 15.67 min C₁₈ 5 µm 100 Å Kromasil AIT column, 4.6x150 mm, λ 230 nm), eluted with a 0-30% linear gradient over 20 min f CH₃CN in 0.01 M aq. TFA at 1.0 mL.min⁻¹. ESI-MS for C₄₈H₇₈O₂₅N₄S₂ (M, 1174.43), found *m*/*z* 1175.5 [M+H]⁺.

Sulfo-LC-SPDP (2.0 mg, 1.8 equiv) was added to a solution of synthetic [AB(E)CD]₃ bearing an aminoethyl aglycon [23] (5.4 mg, 2.2 µmol) in 500 µL PBS pH 7.3 and the mixture was stirred for 1 h at room temperature. RP-HPLC purification of the crude material (C₁₈ 5 µm 100 Å Kromasil AIT column, 10x250 mm, λ 230 nm) eluted with a 0-30% linear gradient over 20 min of CH₃CN in 0.01 M aq. TFA at 5.5 mL.min⁻¹, followed by freeze-drying, gave [AB(E)CD]₃-S-SPy (3.6 mg, 59%) as a lyophilized powder. Rt (HPLC): 14.78 min (C₁₈ 5 µm 100 Å Kromasil AIT column, 4.6x150 mm, λ 230 nm) eluted with a 0-30% linear gradient over 20 min of CH₃CN in 0.01 M aq. TFA at 1.0 mL.min⁻¹. ESI-HRMS for C₁₁₂H₁₈₄O₆₉N₆S₂ ([M+2H]²⁺, 1391.5336), found *m*/*z* 1391.5359 [M+2H]²⁺_{.}

Before coupling to preformed liposomes the thiol functionalized oligosaccharides, AB(E)CD-SH and [AB(E)CD]₃-SH were generated by reacting AB(E)CD-S-S-Py and [AB(E)CD]₃-S-S-Py, respectively, with 1.5 mol eq of tris(2-carboxyethyl)phosphine (TCEP) in 10 mM Hepes buffer containing 145 mM NaCl, pH 6.5 (Buffer A) for 15 min at room temperature.

### 1.2. Synthetic peptide functionalized for coupling

Peptide HA 307-319 bearing an additional cystein at its C-terminus (PKYVKQNTLKLAT-C) (SEQ ID NO: 1) was obtained from NeoMPS (Strasbourg, France). It was reacted before the coupling step with 0.7 mol eq of TCEP in MiMiQ water under the same conditions as above to reduce the putative disulfide bridges.

### 1.3. Preparation of liposomes

PC, PG and Chol (75/20/50 molar ratio) in chloroform/methanol (9/1, v/v) were mixed with Pam₃CAG-Mal (5 mol% of the phospholipids) in a roundbottom flask. After solvent evaporation to dryness under vacuum, the lipid film was rehydrated with Buffer A to obtain a final concentration of 10 mM lipids. The mixture was vortexed and the obtained suspension of multilamellar vesicles was sonicated at room temperature for 1 h under a continuous flow of argon using a 3-mm diameter probe sonicator (Vibra Cell, Sonics and Material Inc., Danubry, CT, USA) at 300W pulsed mode (5 s cycles interrupted for 1.25 s). SUV (Small Unilamellar Vesicles) preparations were finally obtained and centrifuged at 10,000*g* to remove the titanium probe fragments. Vesicle size was determined by dynamic light scattering using a Zeta Master 3000 apparatus (Malvern Instruments, Paris, France). The vesicles were uniformly distributed in size and the mean diameter ranged between 70-90 nm.

### 1.4. Coupling of B-cell and Th epitopes to liposomes

Construct **1:** The functionalized pentasaccharide B-cell epitope AB(E)CD-SH was conjugated to the surface of liposomes containing the Pam₃CAG-Mal anchor. In a typical experiment, 0.125 µmol of freshly prepared AB(E)CD-SH (0.5 mol eq. *vs*. surface-accessible maleimide functions) were added to liposomes (10 µmol phospholipids) in 1 mL Buffer A. After 1 h at room temperature under gentle stirring and argon atmosphere, 0.125 µmol of Th epitope (PKYVKQNTLKLAT-C) previously reduced with TCEP (as described above) was subsequently added. After an additional 1 h, a 10-fold excess of 2-mercaptoethanol was added to inactivate all the unreacted maleimide groups.

Construct **2:** The liposomal construct containing [AB(E)CD]₃ was prepared similarly. However, to keep the AB(E)CD surface density identical to Construct 1 (see above), 0.042 µmol of [AB(E)CD]₃-SH (0.166 mol eq. *vs*. surface-accessible maleimide functions) was used. The amount of Th epitope was kept identical in both constructs.

Construct **3:** A control construct lacking the B-cell epitope was prepared by coupling 0.5 mol eq. of the peptide HA 307-319 in a 10 mM Hepes buffer (pH 6.5), containing 5% (w/v) sorbitol, and the unreacted maleimide was reacted as above with a 10-fold excess of 2-mercaptoethanol.

At 30 min post-addition of the thiol scavenger to each of the three constructs, the liposomal preparations were dialyzed (MW cut-off 10-12 kDa) extensively against 10 mM Hepes buffer (pH 7.4) containing 5% (w/v) sorbitol (Buffer B) to remove unconjugated epitopes and excess reagents. Phospholipid quantification was performed using a colorimetric enzymatic method (Phospholipids Kit B, Wako Chemicals, Neuss, Germany). Aliquots of the liposomal constructs (1-5 µL) were added in triplicates to 96-well microplates and allowed to react with 200 µL of the reagent prepared according to the manufacturer's instructions. The plates were then incubated at 37 °C for 10 min and the optical density was read at 490 nm on a microplate reader (Bio-Rad, model 550). The size of the constructs measured after the conjugation steps displayed an average diameter ranging between 100 and 120 nm. The preparations were frozen at -20 °C after a further addition of sorbitol (10% w/v final concentration).

### 1.5. Quantification of oligosaccharides and peptides conjugated to liposomes

Oligosaccharides AB(E)CD and [AB(E)CD]₃ coupled to the surface of the liposomes were quantified using an adapted Monsigny's method [32]. Importantly, before reading the optical density at 415 nm, the samples were filtered either through PTFE filters (0.45 µm) or by centrifugation during 10 min at 1,500g on filter plates (Acroprep^{™}, 1 µm, A/B glass, NTRL; PALL, Saint-Germain-en-Laye, France). Peptide quantification was achieved after acid hydrolysis of the constructs using Böhlen's fluorometric method [33] previously adapted to liposomal formulations containing peptide epitopes [18]. The signal of the glycoliposomes lacking the Th peptide, was measured under the same experimental conditions, and subtracted from the total fluorescence of the final constructs to deduce the amount of covalently linked peptides.

### 1.6. Immunogenicity of the liposomal constructs

The liposomal formulations were unfrozen and slightly sonicated (5 min) with a sonicator probe to break any aggregates and to recover their original size. Seven-week old female BALB/c mice were immunized intramuscularly (i.m.) 3 times at 3 weeks intervals, followed by a fourth injection (boost) 1 month after the third one. Each mouse received 100 µL of the liposomal construct per injection corresponding, for Constructs **1** and **2,** to an equivalent of 10 µg of AB(E)CD or [AB(E)CD]₃ oligosaccharides and 20 µg of peptide. Control mice received Construct **3** (18 µg of peptide/mouse/injection).

For the AB(E)CD-liposomal constructs (Construct **1**), an experiment including 6 mice was performed. For Construct **2** incorporating [AB(E)CD]₃, three independent experiments, involving different glycoliposome preparations, were performed using 6, 15, and 14 mice, respectively. Control experiments using Construct 3 were performed twice, using 4 and 6 mice, respectively.

Mice were bled 1 week after each immunization, and 4 months after the boost. The IgM and IgG Ab response against the biotinylated oligosaccharides [24] and SF2a LPS was measured by ELISA as previously described [24, 26]. The Ab titer was defined as the dilution of immune serum that gave an OD at least twice that observed with pre-immune serum.

### 1.7. Protective capacity of the construct-induced anti-LPS 2a IgG

Passive immunization was performed as previously described [26]. Briefly, mouse sera exhibiting a similar anti-LPS 2a Ab titer (1/12,800 or 1/6,400) were selected from each group of mice immunized with Construct **2.** The selected immune sera (15 µL) were mixed with 10⁶ CFU of virulent SF2a bacteria (5 µL) just before i.n. administration of the total 20 µL to naive mice. Measurement of lung-bacterial load (CFU/lungs) was done at 24 h post-infection. A control group was included in which naive mice received SF2a bacteria previously incubated with pre-immune serum. Three independent experiments including 7 mice per group were run for each immune serum tested.

### Example 2. Results

### 2.1. Preparation and characterization of the liposomal constructs

The liposomal constructs used in this study contained the thiol-reactive adjuvant derivative Pam₃CAG-Mal (Fig. 2A) [17]. It serves as anchor for both the B-cell and Th epitopes. Accordingly, the oligosaccharides AB(E)CD and [AB(E)CD]₃, bearing an aminoethyl aglycon [22, 23], i.e. the B-cell epitopes corresponding respectively to one and three RUs of SF2a O-SP, were functionalized with a heterobifunctional reagent Sulfo-LC-SPDP to allow their conjugation to Pam₃CAG-Mal after reduction of the disulfide bond (Fig. 1B). Similarly, hemagglutinin influenza peptide HA 307-319, a "promiscuous" Th epitope [27, 28] was elongated at its C-terminus with a cysteine residue (PKYVKQNTLKLAT-C) suitable for coupling.

The conjugation of the SH-modified B-cell and Th epitopes (Figs. 1 and 2) to preformed liposomes could be performed under mild conditions in a saline buffer (Fig. 2B) and three liposomal constructs **(1-3)** were prepared (Fig. 3). Constructs **1** and **2** carry HA 307-319 and AB(E)CD or [AB(E)CD]₃, respectively. As a control, in Construct 3 the B-cell epitope was replaced by 2-mercaptoethanol. Importantly, the relative molar quantities of conjugated AB(E)CD and [AB(E)CD]₃ were chosen to maintain at the surface of the liposomal constructs an AB(E)CD density that was independent of the molecular weight of the coupled oligosaccharide. The inventors observed that the reaction order was of great importance to achieve nearly quantitative coupling yields for both oligosaccharides and peptide and to avoid vesicle aggregation, i.e. oligosaccharides were conjugated first, followed by peptide HA 303-319. Although the inventors did not study this aspect further, a tentative explanation can be provided: in a saline buffer, the presence of the conjugated oligosaccharides maintains a hydrophilic/polar environment on the outer surface of the vesicles, thus limiting the interactions between the more hydrophobic peptide epitopes which could favor aggregation. The mean size of the vesicles was measured prior and after coupling; all preparations were uniformly distributed with a diameter ranging between 70 and 90 nm. The vesicles size remained unaffected upon epitope coupling in Constructs **1** and **3.** However, a slight increase was found for Construct **2,** whose diameter reached 100-120 nm in average.

### 2.2. Immunogenicity of the liposomal constructs

To assess the immunogenicity of Constructs **1** and **2,** BALB/c mice were immunized intramuscularly (i.m.) using an equivalent of 10 µg of oligosaccharide per dose. Ab detection was performed 7 days after the second, third and fourth injections. No toxicity of the constructs was noted at the dose used. Construct **1,** carrying AB(E)CD i.e. one SF2a O-SP RU, elicited neither anti-LPS 2a nor anti-AB(E)CD Ab responses (data not shown). In contrast, as shown in Fig. 4, an Ab response was raised with Construct **2** carrying [AB(E)CD]₃ i.e. three SF2a O-SP RUs. In two independent experiments, the responding mice were about 93%. The mean IgG titers (Fig. 4A and 4B) increased from the second to the third immunization and after the boost for both anti-LPS 2a and anti-[AB(E)CD]₃ IgG responses. An important heterogeneity in the immune response from one mouse to another was noticed. No Ab response against LPS 2a and the two oligosaccharide haptens was detected in sera of control mice vaccinated with Construct **3** (data not shown). Taken together, these results clearly show that the liposomal construct carrying [AB(E)CD]₃ is immunogenic in mice, triggering the induction of Abs not only directed against the synthetic oligosaccharide but also against the native SF2a LPS.

### 2.3. Immune response profile

To further detail the Ab response induced by Construct **2,** IgM and IgG subclasses Ab responses to SF2a LPS were measured in a set of sera selected after the boost. As shown in Fig. 5, the IgG Ab response prevailed over that of IgM. Amongst the IgG subclasses, IgG1 and IgG3 were predominantly induced. It is noteworthy that four months after the final boost anti-LPS 2a and anti-[AB(E)CD]₃ Abs were still detected with an average Ab titer that was 3- to 4-fold less than that measured after the boost. An additional boost given at that time resulted in a two-fold increase of the Ab titer one week later (data not shown). Altogether, these results show that the liposome-based presentation of [AB(E)CD]₃ triggers a Th2-type, long-lasting anti-LPS 2a Ab response, suggesting the induction of a memory response.

### 2.4. Protective capacity of the anti-LPS 2a Abs induced by the liposomal constructs

To assess the protective efficacy of the anti-LPS 2a Abs induced upon immunization with Construct **2,** naive mice were passively i.n. administered with immune or pre-immune sera previously incubated with virulent SF2a bacteria. A positive control using sera from mice immunized with [AB(E)CD]₃-TT [26] was included. Immune sera with the same anti-LPS 2a Ab titer, i.e. 1/6400, were selected for each construct. Protection was evaluated by measuring the CFU/lungs at 24 h post-infection and a reduction factor was calculated as the ratio between CFU/lungs in naive mice passively administered with pre-immune sera to that of naive mice passively administered with immune sera. Although less efficient compared to [AB(E)CD]₃-TT, the Construct **2**-induced anti-LPS 2a Abs were also able to limit the infection. Indeed, as shown in Fig. 6, the reduction factor measured was 11, meaning a reduction of 11-fold the CFU counts within the lungs of mice passively administered with those Abs as compared to control mice receiving the pre-immune sera. Protection was dose-dependent since the reduction factor was 4.4 times lower with Construct **2-**induced sera diluted 10 times prior to its administration in combination with virulent bacteria. Altogether these results indicate that presentation of [AB(E)CD]₃ via the diepitope liposomal Construct **2** allows the induction of protective anti-LPS 2a Ab.

### References

[1] Kelly DF, Moxon ER, Pollard AJ. Haemophilus influenzae type b conjugate vaccines. Immunology 2004;113(2):163-74.
[2] Rappuoli R. Conjugates and reverse vaccinology to eliminate bacterial meningitis. Vaccine 2001;19(17-19):2319-22.
[3] Pozsgay V. Recent developments in synthetic oligosaccharide-based bacterial vaccines. Curr Top Med Chem 2008;8(2):126-40.
[4] Verez-Bencomo V, Fernandez-Santana V, Hardy E, Toledo ME, Rodriguez MC, Heynngnezz L, et al. A synthetic conjugate polysaccharide vaccine against Haemophilus influenzae type b. Science 2004;305(5683):522-5.
[5] Schutze MP, Leclerc C, Jolivet M, Audibert F, Chedid L. Carrier-induced epitopic suppression, a major issue for future synthetic vaccines. J Immunol 1985;135(4):2319-22.
[6] Baraldo K, Mori E, Bartoloni A, Norelli F, Grandi G, Rappuoli R, et al. Combined conjugate vaccines: enhanced immunogenicity with the N19 polyepitope as a carrier protein. Infect Immun 2005;73(9):5835-41.
[7] Shahiwala A, Vyas TK, Amiji MM. Nanocarriers for systemic and mucosal vaccine delivery. Recent Pat Drug Deliv Formul 2007;1(1):1-9.
[8] Alving CR. Liposomes as carriers of antigens and adjuvants. J Immunol Methods 1991;140(1):1-13.
[9] Gregoriadis G. Engineering liposomes for drug delivery: progress and problems. Trends Biotechnol 1995;13(12):527-37.
[10]Fernandes I, Frisch B, Muller S, Schuber F. Synthetic lipopeptides incorporated in liposomes: in vitro stimulation of the proliferation of murine splenocytes and in vivo induction of an immune response against a peptide antigen. Mol Immunol 1997;34(8-9):569-76.
[11]Friede M, Muller S, Briand JP, Van Regenmortel MH, Schuber F. Induction of immune response against a short synthetic peptide antigen coupled to small neutral liposomes containing monophosphoryl lipid A. Mol Immunol 1993;30(6):539-47.
[12]Roth A, Espuelas S, Thumann C, Frisch B, Schuber F. Synthesis of thiol-reactive lipopeptide adjuvants. Incorporation into liposomes and study of their mitogenic effect on mouse splenocytes. Bioconjug Chem 2004;15(3):541-53.
[13]Fortin A, Lagace J, Therien HM. Trafficking of surface-linked and encapsulated liposomal antigens in macrophages: an immunocytochemical study. J Histochem Cytochem 2001;49(11):1407-20.
[14]Nair S, Buiting AM, Rouse RJ, Van Rooijen N, Huang L, Rouse BT. Role of macrophages and dendritic cells in primary cytotoxic T lymphocyte responses. Int Immunol 1995;7(4):679-88.
[15]Grivel JC, Crook K, Leserman L. Endocytosis and presentation of liposome-associated antigens by B cells. Immunomethods 1994;4(3):223-8.
[16]Puffer EB, Pontrello JK, Hollenbeck JJ, Kink JA, Kiessling LL. Activating B cell signaling with defined multivalent ligands. ACS Chem Biol 2007;2(4):252-62.
[17]Boeckler C, Dautel D, Schelte P, Frisch B, Wachsmann D, Klein JP, et al. Design of highly immunogenic liposomal constructs combining structurally independent B cell and T helper cell peptide epitopes. Eur J Immunol 1999;29(7):2297-308.
[18]Roth A, Rohrbach F, Weth R, Frisch B, Schuber F, Wels WS. Induction of effective and antigen-specific antitumour immunity by a liposomal ErbB2/HER2 peptide-based vaccination construct. Br J Cancer 2005;92(8):1421-9.
[19]Levine M M, Kotloff KL, Barry EM, Pasetti MF, Sztein MB. Clinical trials of Shigella vaccines: two steps forward and one step back on a long, hard road. Nat Rev Microbiol 2007;5(7):540-53.
[20]Passwell JH, Harlev E, Ashkenazi S, Chu C, Miron D, Ramon R, et al. Safety and immunogenicity of improved Shigella O-specific polysaccharideprotein conjugate vaccines in adults in Israel. Infect Immun 2001;69(3):1351-7.
[21]Fries LF, Montemarano AD, Mallett CP, Taylor DN, Hale TL, Lowell GH. Safety and immunogenicity of a proteosome-Shigella flexneri 2a lipopolysaccharide vaccine administered intranasally to healthy adults. Infect Immun 2001;69(7):4545-53.
[22]Wright K, Guerreiro C, Laurent I, Baleux F, Mulard LA. Preparation of synthetic glycoconjugates as potential vaccines against Shigella flexneri serotype 2a disease. Org Biomol Chem 2004;2(10):1518-27.
[23]Bélot F, Guerreiro C, Baleux F, Mulard LA. Synthesis of two linear PADRE conjugates bearing a deca- or pentadecasaccharide B epitope as potential synthetic vaccines against Shigella flexneri serotype 2a infection. Chemistry 2005;11(5):1625-35.
[24]Phalipon A, Costachel C, Grandjean C, Thuizat A, Guerreiro C, Tanguy M, et al. Characterization of functional oligosaccharide mimics of the Shigella flexneri serotype 2a O-antigen: implications for the development of a chemically defined glycoconjugate vaccine. J Immunol 2006;176(3):1686-94.
[25]Vulliez-Le Normand B, Saul FA, Phalipon A, Belot F, Guerreiro C, Mulard LA, et al. Structures of synthetic O-antigen fragments from serotype 2a Shigella flexneri in complex with a protective monoclonal antibody. Proc Natl Acad Sci U.S.A. 2008;105(29):9976-81.
[26]Phalipon A, Tanguy M, Grandjean C, Guerreiro C, Belot F, Cohen D, et al. A synthetic carbohydrate-protein conjugate vaccine candidate against Shigella flexneri 2a infection. J Immunol 2009;182(4):2241-7.
[27] Falugi F, Petracca R, Mariani M, Luzzi E, Mancianti S, Carinci V, et al. Rationally designed strings of promiscuous CD4(+) T cell epitopes provide help to Haemophilus influenzae type b oligosaccharide: a model for new conjugate vaccines. Eur J Immunol 2001;31(12):3816-24.
[28]Baraldo K, Mori E, Bartoloni A, Petracca R, Giannozzi A, Norelli F, et al. N19 polyepitope as a carrier for enhanced immunogenicity and protective efficacy of meningococcal conjugate vaccines. Infect Immun 2004;72(8):4884-7.
[29]Reitermann A, Metzger J, Wiesmuller KH, Jung G, Bessler WG. Lipopeptide derivatives of bacterial lipoprotein constitute potent immune adjuvants combined with or covalently coupled to antigen or hapten. Biol Chem Hoppe Seyler 1989;370(4):343-52.
[30] Takeuchi O, Sato S, Horiuchi T, Hoshino K, Takeda K, Dong Z, et al. Cutting edge: role of Toll-like receptor 1 in mediating immune response to microbial lipoproteins. J Immunol 2002; 169(1):10-4.
[31]Deiters U, Barsig J, Tawil B, Muhlradt PF. The macrophage-activating lipopeptide-2 accelerates wound healing in diabetic mice. Exp Dermatol 2004;13(12):731-9.
[32]Said Hassane F, Frisch B, Schuber F. Targeted liposomes: convenient coupling of ligands to preformed vesicles using "click chemistry". Bioconjug Chem 2006;17(3):849-54.
[33]Bohlen P, Stein S, Dairman W, Udenfriend S. Fluorometric assay of proteins in the nanogram range. Arch Biochem Biophys 1973;155(1):213-20.
[34]Kaufmann SH. The contribution of immunology to the rational design of novel antibacterial vaccines. Nat Rev Microbiol 2007;5(7):491-504.
[35]Altin JG, Parish CR. Liposomal vaccines-targeting the delivery of antigen. Methods 2006;40(1):39-52.
[36] Schelte P, Boeckler C, Frisch B, Schuber F. Differential reactivity of maleimide and bromoacetyl functions with thiols: application to the preparation of liposomal diepitope constructs. Bioconjug Chem 2000;11(1):118-23.
[37]Wiesmuller KH, Fleckenstein B, Jung G. Peptide vaccines and peptide libraries. Biol Chem 2001;382(4):571-9.
[38]Nardin EH, Calvo-Calle JM, Oliveira GA, Nussenzweig RS, Schneider M, Tiercy JM, et al. A totally synthetic polyoxime malaria vaccine containing Plasmodium falciparum B cell and universal T cell epitopes elicits immune responses in volunteers of diverse HLA types. J Immunol 2001;166(1):481-9.
[39]Mittenbuhler K, v d Esche U, Heinevetter L, Bessler WG, Huber M. Lipopeptides: adjuvanticity in conventional and genetic immunization. FEMS Immunol Med Microbiol 2003;37(2-3):193-200.
[40]Buskas T, Ingale S, Boons GJ. Towards a fully synthetic carbohydrate-based anticancer vaccine: synthesis and immunological evaluation of a lipidated glycopeptide containing the tumor-associated tn antigen. Angew Chem Int Ed Engl 2005;44(37):5985-8.
[41]Ingale S, Wolfert MA, Gaekwad J, Buskas T, Boons GJ. Robust immune responses elicited by a fully synthetic three-component vaccine. Nat Chem Biol 2007;3(10):663-7.
[42]Ingale S, Wolfert MA, Buskas T, Boons GJ. Increasing the antigenicity of synthetic tumor-associated carbohydrate antigens by targeting Toll-like receptors. ChemBioChem 2009;10(3):455-63.
[43]Toyokuni T, Hakomori S, Singhal AK. Synthetic carbohydrate vaccines: synthesis and immunogenicity of Tn antigen conjugates. Bioorg Med Chem 1994;2(11):1119-32.
[44]Toyokuni T, Dean B, Cai S, Boivin D, Hakomori S, Singhal AK. Synthetic vaccines : Synthesis of a dimeric Tn antigen-lipopeptide conjugate that elicits immune responses against Tn-expressing glycoproteins. J Am Chem Soc 1994;116:395-96.
[45] Reichel F, Ashton PR, Boons GJ. Synthetic carbohydrate-based vaccines : synthesis of an L-glycero-D-manno-heptose antigen-T-epitope-lipopeptide conjugate. Chem Commun 1997:2087-88.
[46] Bundle DR. A carbohydrate vaccine exceeds the sum of its parts. Nat Chem Biol 2007;3(10):605-6.
[47] Shahum E, Therien HM. Liposomal adjuvanticity: effect of encapsulation and surface-linkage on antibody production and proliferative response. Int J Immunopharmacol 1995;17(1):9-20.
[48] Therien HM, Lair D, Shahum E. Liposomal vaccine: influence of antigen association on the kinetics of the humoral response. Vaccine 1990;8(6):558-62.
[49]BenMohamed L, Wechsler SL, Nesburn AB. Lipopeptide vaccines- yesterday, today, and tomorrow. Lancet Infect Dis 2002;2(7):425-31.
[50] Snippe H, van Dam JE, van Houte AJ, Willers JM, Kamerling JP, Vliegenthart JF. Preparation of a semisynthetic vaccine to Streptococcus pneumoniae type 3. Infect Immun 1983;42(2):842-4.
[51]Zigterman G, Verheul A, Snippe H. Liposomes as immunadjuvants for saccharide antigens. Liposome in Drug Delivery by G Gregoriadis, AT Florence, HM Patel Eds 1993 Harwood Academic Publisher (Chur, Switzerland).
[52] Robin G, Cohen D, Orr N, Markus I, Slepon R, Ashkenazi S, et al. Characterization and quantitative analysis of serum IgG class and subclass response to Shigella sonnei and Shigella flexneri 2a lipopolysaccharide following natural Shigella infection. J Infect Dis 1997;175(5):1128-33.
[53]Robin G, Keisari Y, Slepon R, Ashkenazi S, Cohen D. Quantitative analysis of IgG class and subclass and IgA serum response to Shigella sonnei and Shigella flexneri 2a polysaccharides following vaccination with Shigella conjugate vaccines. Vaccine 1999;17(23-24):3109-15.
[54] Slack J, Der-Balian GP, Nahm M, Davie JM. Subclass restriction of murine antibodies. II. The IgG plaque-forming cell response to thymus-independent type 1 and type 2 antigens in normal mice and mice expressing an X-linked immunodeficiency. J Exp Med 1980;151(4):853-62.
[55] Stein KE. Thymus-independent and thymus-dependent responses to polysaccharide antigens. J Infect Dis 1992;165:S49-552.
[56] D. Cohen, M. S. Green, C. Block, T. Rouach and I. Ofek, J. Infect. Dis., 1988, 157, 1068-1071.
[57] D. Cohen, M. S. Green, C. Block, R. Slepon and I. Ofek, J. Clin. Microbiol., 1991, 29, 386-389.
[58] Pirofski, L.A., Trends in microbiology 9, 445-451 (2001).
[59]Nyame, A.K., et al., Archives of biochemistry and biophysics 426, 182-200 (2004).
[60] Ragupathi, G., Cancer Immunol Immunother 43, 152-157 (1996).
[61] Boeckler, C., Frisch, B., Schuber, F., Bioorganic & Medicinal Chemistry Letters 8 (1998) 2055-2058.
[62] WO 2007/079448
[63] Gololobov, Y. G. (1981), "Sixty years of staudinger reaction", Tetrahedron 37: 437
[64] Kolb H. C., Finn M. G. and Sharpless K. B. (2001). "Click Chemistry: Diverse Chemical Function from a Few Good Reactions". Angewandte Chemie International Edition 40 (11): 2004-2021.
[65] Dawson PE, Muir TW, Clark-Lewis I, Kent, SBH. 1994. Synthesis of Proteins by Native Chemical Ligation. Science 266:776-779.
[66] J Alexander et al. Immunity, Vol. 1 , 751 -761 , 1994.
[67] Weiner GJ, Journal od Leukocyte Biology, Vol. 68, October 2000.
[68] Weiner GJ et al, PNAS, Vol. 94, pp. 10833-10837, September 1997.
[69] O'Sullivan D, Arrhenius T, Sidney J, Del Guercio MF, Albertson M, Wall M, Oseroff C, Southwood S, Colón SM, Gaeta FC, et al. On the interaction of promiscuous antigenic peptides with different DR alleles. Identification of common structural motifs. J Immunol. 1991 Oct 15;147(8):2663-9.
[70] Montalbetti C. A. G. N., and Falque V. Amide bond formation and peptide coupling, Tetrahedron 61 (2005) 10827-10852

## Claims

1. A diepitope construct comprising:
a liposome, comprising a plurality of functionalized lipid anchors disposed upon a surface thereof;
a microbial saccharide epitope, comprising a first chemical attachment moiety;
a peptide epitope, comprising a second chemical attachment moiety,
wherein said saccharide epitope and said peptide epitope are not covalently attached to each other; and
wherein said saccharide epitope and said peptide epitope are covalently attached to said plurality of functionalized lipid anchors.

2. The diepitope construct according to claim 1, wherein said saccharide epitope is a B cell epitope and said peptide epitope is a Th cell epitope.

3. The diepitope construct according to claim 1 or 2, wherein said saccharide epitope is synthetic.

4. The diepitope construct according to any one of claims 1 to 3, wherein said saccharide epitope comprises multiple saccharide units.

5. The diepiptope construct according to any one of claims 1 to 4, wherein said first chemical attachment moiety comprises a thiol precursor group.

6. The diepitope construct according to claim 5, wherein said plurality of functionalized liposome anchors comprise a thiol-reactive functional group.

7. The diepitope construct according to claim 6, wherein said thiol-reactive functional group is a maleimide group.

8. The diepitope construct wherein said plurality of lipid anchors are adjuvants.

9. The diepitope construct according to claim 7 or 8, wherein said plurality of lipid anchors consist of S-[2,3-bis(palmitoyloxy)-2(R)-propyl]-N-palmitoyl-(R)-cysteinyl-alanyl-glycine-maleimide (Pam₃CAG-Mal).

10. The diepitope construct according to any one of claims 1 to 7, wherein said plurality of functionalized lipid anchors comprise a first population of functionalized lipid anchors which are not adjuvants and a second population of lipid anchors which are adjuvants.

11. The diepitope construct according to any one of claims 1 to 10, wherein said second chemical attachment moiety comprises at least one cysteine residue.

12. A method to create a diepitope liposome construct according to any one of claims 1 to 11, comprising the steps:
a. preparing at least one set of liposomes, wherein each liposome comprises at least two liposome anchors;
b. preparing at least one set of saccharide epitopes and at least one set of peptide epitopes;
c. chemically activating said liposome anchors and said epitopes from steps a;.
d. mixing said liposomes and said epitopes from step c;
e. chemically activating said epitopes from step b and mixing with said liposomes from step d.

13. Use of a liposome according to anyone of claims 1 to 11, or made using a method according to claim 12 or a saccharide consisting of the formula AB(E)CD-SH, wherein A is an α-L-rhamnopyranosyl-(1,2) residue, B is an α-L-rhamnopyranosyl-(1,3) residue, C is an α-L-rhamnopyranosyl-(1,3) residue, E is an α-D-glucopyranosyl-(1,4) residue, D is a 2-acetamido-2-deoxy-β-D-glucopyranosyl-(1,2) residue and -SH is a thiol functional group for the preparation of a medicament.

14. A vaccine comprising a liposome according to anyone of claims 1 to 11, or made using a method according to claim 12 or a saccharide consisting of the formula AB(E)CD-SH, wherein A is an α-L-rhamnopyranosyl-(1,2) residue, B is an α-L-rhamnopyranosyl-(1,3) residue, C is an α-L-rhamnopyranosyl-(1,3) residue, E is an α-D-glucopyranosyl-(1,4) residue, D is a 2-acetamido-2-deoxy-β-D-glucopyranosyl-(1,2) residue and -SH is a thiol functional group.
